Europäisches Patentamt

European Patent Office

Office européen des brevets

(11) Veröffentlichungsnummer: 0 170 031
A1

(19)

(12) # EUROPÄISCHE PATENTANMELDUNG

(21) Anmeldenummer: 85107615.8

(22) Anmeldetag: 20.06.85

(51) Int. Cl.⁴: A 61 K 49/02

(30) Priorität: 04.07.84 CH 3222/84

(43) Veröffentlichungstag der Anmeldung:
05.02.86 Patentblatt 86/6

(84) Benannte Vertragsstaaten:
AT BE DE FR GB IT NL SE

(71) Anmelder: Gesellschaft zur Förderung der
industrieorientierten Forschung an den Schweizerischen
Hochschulen und weiteren Institutionen
Wildhainweg 21
CH-3012 Bern(CH)

(72) Erfinder: Häberli, André, Dr.
Weiherstrasse 9
CH-3073 Gümligen(CH)

(72) Erfinder: Schubiger, P. August, Dr.
Holegrabestrasse 589 b
CH-5426 Lengnau(CH)

(74) Vertreter: Scheidegger, Zwicky, Werner & Co.
Stampfenbachstrasse 48 Postfach
CH-8023 Zürich(CH)

(54) Radioaktives Diagnosemittel zum Nachweisen einer venösen Thrombose.

(57) Ein intravenös injizierbares Diagnosemittel zum Nachweisen einer venösen Thrombose enthält ein mit einem Gamma-oder Positronenstrahler markiertes Fragment von menschlichem Plaminogen, z.B. $^{123}$I-Neoplasmin. Das in die Venen eingebrachte radioaktiv markierte Plasminogenfragment sammelt sich am Ort eines Thrombus an, so dass dort eine erhöhte radioaktive Strahlung entsteht, die durch eine der bekannten nuklear medizinischen Techniken schon 15 bis 30 Minuten nach der Injektion des Diagnosemittels feststellbar ist.

Zur Herstellung des Diagnosemittels wird menschliches Plasminogen mit Elastase gespalten und aus dem Reaktionsgemisch Neoplasminogen gewonnen. Letzteres wird anschliessend mit einem radioaktiven Halogen oder Metallion derart markiert, dass eine chemische Bindung zwischen dem radioaktiven Atom und freien Tyrosingruppen bzw. Aminogruppen des Neoplasminogens herbeigeführt wird. Erst etwa 15 Minuten vor der intravenösen Injektion wird das markierte Neoplasminogen durch Zugabe von Urokinase biologisch aktiviert und damit die Bildung von radioaktiv markiertem Neoplasmin eingeleitet.

Mit diesem Diagnosemittel ist nicht nur eine rasche, nicht invasive und verhältnismässig treffsichere Diagnostizierung einer Thrombose ermöglicht, sondern auch erreicht, dass bein dem nachfolgenden Abbau im menschlichen Organismus keine unvorhergesehenen Nebenwirkungen auftreten.

0170031

Radioaktives Diagnosemittel zum Nachweisen einer
venösen Thrombose

_____

Die vorliegende Erfindung betrifft ein intravenös injizierbares radioaktives Diagnosemittel zum Nachweisen einer venösen Thrombose durch nuklearmedizinische Techniken, welches
Diagnosemittel ein mit einem radioaktiven Atom markiertes
Fragment von menschlichem Plasminogen aufweist. Ferner betrifft die Erfindung ein Verfahren und ein Präparat zur Herstellung eines solchen Diagnosemittels.

Ohne Zweifel besteht das Bedürfnis, das Vorliegen einer venösen Thrombose mit hoher Sicherheit und in verhältnismässig
kurzer Zeit feststellen oder ausschliessen zu können, ohne dabei die zu untersuchende Person durch Diagnosemittel und Bestrahlungen erheblich belasten zu müssen. Die bisher bekannten Diagnoseverfahren waren wenig spezifisch, zeitraubend,
invasiv oder mit der Gefahr von gesundheitsstörenden Nebenwirkungen verbunden.

Eine invasive und für den Patienten schmerzhafte Untersuchung
ist die Phlebographie oder Venographie, bei welcher in die
Venen ein radiographisches Kontrastmittel eingebracht und unmittelbar anschliessend von den zu untersuchenden Körperteilen
Röntgenbilder erzeugt werden, welche den gegebenenfalls vorhandenen Thrombus erkennen lassen.

Zu den wenig spezifischen und daher unsicheren Diagnoseverfahren gehören die auf örtlichen Temperaturunterschieden beruhende Thermographie, die auf der elektrischen Leitfähigkeit des Blutes beruhende Plethysmographie und die auf Schallwellenreflexionen beruhende Ultraschalluntersuchung.

Verhältnismässig sichere Resultate liefert der Radiofibrinogen-Test, bei welchem mit dem Isotop I-125 markiertes mensch-

liches Fibrinogen intravenös injiziert wird, wonach am Ort
des gegebenenfalls vorhandenen Thrombus das markierte Fibrinogen zusammen mit dem im menschlichen Körper vorhandenen
natürlichen Fibrinogen zu Fibrin abgebaut wird. Dabei entsteht am Ort des Thrombus eine höhere Konzentration des Isotopes, was örtlich eine erhöhte radioaktive Strahlung zur
Folge hat, die durch vergleichende Aktivitätsmessungen an der
Oberfläche des zu untersuchenden Körperteiles des Probanden
feststellbar und lokalisierbar ist. Nachteilig bei diesem
Diagnoseverfahren ist, dass zwischen der Injektion des radioaktiv markierten Fibrinogens und den Oberflächenaktivitätsmessungen 6 bis 24 Stunden gewartet werden muss, da die natürliche Bildung des Fibrins und damit die Anlagerung des
Isotopes am Ort des Thrombus nur verhältnismässig langsam erfolgt. Die gleiche Wartezeit ist auch notwendig bei Durchführung eines Scintigraphie-Tests, bei welchem mit I-131 oder
I-123 markiertes Fibrinogen intravenös injiziert wird und
nach Ablauf der erwähnten Wartefrist Abbildungen der Blutbahnen in dem zu untersuchenden Körperteil mittels einer
Scintillationskamera erzeugt werden, wobei die örtliche Verteilung des Isotopes ersichtlich wird.

Ein anderer bekannter Test beruht auf der Erkenntnis, dass
am Ort eines Blutgerinsels auf natürliche Weise Plasmin, d.h.
ein Fragment von Plasminogen, gebildet wird, das im menschlichen Blut in einer Menge von etwa 0,1 g pro Liter Blut vorhanden ist. Das Plasmin ist imstande, das bei der Blutgerinnung entstehende Fibringerinsel zu "Spaltpeptiden" abzubauen
und damit das Blutgerinsel allmächlich aufzulösen. Es wurde
erkannt, dass zur Diagnose einer venösen Thrombose dem Patienten ein mit einem radioaktiven Atom markiertes Fragment
von menschlichem oder tierischem Plasminogen intravenös verabreicht werden kann und dieses Plasminogenfragment sich am
Ort des gegebenenfalls vorhandenen Thrombus wie natürliches
Plasmin ansammelt, wonach mittels nuklearmedizinischer Techniken eine erhöhte Aktivität im Bereich des Thrombus feststellbar ist und der Thrombus lokalisiert werden kann. Im Gegensatz zu dem vorher erwähnten Fibrinogen-Test beträgt in diesem Fall die notwendige Wartezeit zwischen der intravenösen Injektion und den Aktivitätsmessungen weniger als eine Stunde, so dass gegebenenfalls dem Patienten rechtzeitig ein blutverdünnendes Mittel verabreicht werden kann, bevor sich Folgekomplikationen, wie z.B. eine Lugenembolie, einstellen. Diese vorteilhafte rasche Wirkung des radioaktiven Plasminogenfragmentes hat ihren Grund vermutlich darin, dass dieses Diagnosemittel sich am Ort einer Thrombose gleich verhält wie natürliches Plasmin, wobei aber die für die Spaltung von Plasminogen zu Plasmin im menschlichen Körper erforderliche Zeitspanne übersprungen wird, da bei diesem Test bereits abgespaltetes Neoplasmin in die Venen eingeführt wird.

Als radioaktives Atom zur Markierung des Plasminogenfragmentes hat man bisher Tc-99m verwendet, wobei gemäss einem von R.B.R. Persson und L. Darte im Int. Journal of Applied Radiation and Isotopes, Vol. 28 (1977) p. 97 beschriebenes Verfahren vorgegangen wurde. Bei diesem Verfahren wird das Plasminogenfragment oder Plasmin mit einer NaCl-Lösung von Tc-99m-Pertechnetat und Zinnchlorid als Reduktionsmittel gemischt und diese Mischung während etwa 1 Stunde bei Raumtemperatur stehen gelassen. Dabei wird kolloidales $TcO_2$ gebildet und durch Adsorption an irgendeiner unbestimmten Stelle des Plasmins angeheftet. Das auf diese Weise markierte Plasminogenfragment ist zwar als intravenös zu injizierendes Diagnosemittel zum Nachweisen einer Thrombose geeignet, ist jedoch mit dem Nachteil behaftet, dass es sich bei dem nachfolgenden Abbau im menschlichen Organismus nicht wie ein einheitlicher Stoff verhält, was zu unerwünschten und gegebenenfalls die nukleartechnische Aufnahme störenden Nebenwirkungen, insbesondere in der Leber und den Nieren des Patienten, führen kann.

Aufgabe der vorliegenden Erfindung ist es, ein intravenös injizierbares radioaktives Diagnosemittel zum Nachweisen einer venösen Thrombose zur Verfügung zu stellen, das vom menschlichen Organismus wie ein einheitlicher Stoff natürlichen Ursprungs aufgenommen und verarbeitet wird und nicht zu unvorhergesehenen Nebenwirkungen führt.

Das bei der Lösung dieser Aufgabe gefundene intravenös injizierbare radioaktive Diagnosemittel ist dadurch gekennzeichnet, dass als Gamma- oder Positronenstrahler ein radioaktives Halogen kovalent an freie Tyrosingruppen des Plasminogenfragmentes gebunden ist oder ein radioaktives Metallion mittels eines bifunktionellen Chelators kovalent an freie Aminogruppen des Plasminogenfragmentes oder mittels Azokupplung an freie Tyrosingruppen des Plasminogenfragmentes gebunden ist.

Der an das Plasminogenfragment gebundene Gammastrahler kann z.B. I-123, I-131, In-111, Ru-97 oder Tc-99m sein und eine Energie im Bereich von 80 bis 400 keV aufweisen.

Der an das Plasminogenfragment gebundene Positronenstrahler kann z.B. F-18, I-124 oder BR-77 sein.

Das durch die Erfindung geschaffene Verfahren zur Herstellung des erfindungsgemässen Diagnosemittels ist dadurch gekennzeichnet, dass menschliches Plasminogen mit Elastase gespalten und aus dem Reaktionsgemisch Neoplasminogen durch Chromatographie gewonnen wird, dass ein radioaktives Halogen kovalent an freie Tyrosingruppen des Neoplasminogens gebunden wird oder ein radioaktives Metallion mittels eines bifunktionellen Chelators kovalent an freie Aminogruppen des Neoplasminogens oder mittels Azokupplung an freie Tyrosingruppen des Neoplasminogens gebunden wird, und dass das so mit einem Gamma- oder Positronenstrahler markierte Neoplasminogen vor der intravenösen Injektion mit Urokinase weiter gespalten wird zur Bildung von radioaktiv markiertem, intravenös injizierbarem Neoplasmin.

Vorzugsweise wird das Verfahren in der Weise durchgeführt,
dass man Patientendosen, die je mindestens 200 µCi des radioaktiv markierten Neoplasminogens in mindestens 200 µl 0,1 M
Phosphatpuffer mit pH 7,8 enthalten, bildet und einer solchen
Dosis jeweils etwa 15 Minuten vor der intravenösen Injektion
etwa 3000 E Urokinase zufügt.

Ein für die Durchführung des Verfahrens geeignetes Präparat
zur Herstellung des Diagnosemittels ist erfindungsgemäss dadurch gekennzeichnet, dass das vor der intravenösen Injektion
mit Urokinase zu behandelnde Präparat mindestens 200 µCi von
mit einem Gamma- oder Positronenstrahler markiertem menschlichem Neoplasminogen enthält, bei welchem ein radioaktives
Halogen kovalent an freie Tyrosingruppen des Neoplasminogens
gebunden ist oder ein radioaktives Metallion mittels eines
bifunktionellen Chelators kovalent an freie Aminogruppen des
Neoplasminogens oder mittels Azokupplung an freie Tyrosingruppen des Neoplasminogens gebunden ist.

Vorzugsweise werden aus dem mit einem Gamma- oder Positronenstrahler markierten Neoplasminogen handelsfertige sterile und
pyrogenfreie Patientendosen gebildet, die je etwa 250 µg radioaktiv markiertes Neoplasminogen mit einer Aktivität von
mind. 200 µCi in mind. 200 µl 0,1 M Phosphatpuffer mit pH 7,8
enthalten. Einer solchen Patientendosis werden zweckmässig
erst etwa 15 Minuten vor der intravenösen Injektion noch etwa
3000 E Urokinase zugefügt, um das Neoplasminogen biologisch
zu aktivieren und die Bildung von radioaktiv markiertem Neoplasmin herbeizuführen.

Bereits 15 bis 30 Minuten nach der Injektion des Diagnosemittels lässt sich durch eine der bekannten nuklearmedizinischen
Techniken, z.B. durch Messungen der Oberflächenaktivität an
verschiedenen Körperstellen, das Vorliegen oder die Abwesenheit einer venösen Thrombose mit verhältnismässig guter Treffsicherheit nachweisen.

Weitere Einzelheiten der Erfindung ergeben sich aus den Patentansprüchen und aus der nun folgenden Beschreibung, in welcher die Erfindung rein beispielsweise und unter Bezugnahme auf die beigefügten Zeichnungen näher erläutert ist.

Fig. 1 zeigt eine graphische Darstellung der in verschiedenen Zonen längs des rechten bzw. linken Beines eines gesunden Probanden gemessenen Oberflächenaktivitäten, jeweils 15, 30, 60 und 120 Minuten nach der intravenösen Injektion von mit I-123 markiertem Neoplasmin;

Fig. 2 ist eine analoge Darstellung der an einem Patienten mit einer Thrombose im linken Bein gemessenen Oberflächenaktivitäten, ebenfalls jeweils 15, 30, 60 und 120 Minuten nach der intravenösen Injektion von mit I-123 markiertem Neoplasmin.

Beispiel 1:

Für die Herstellung eines intravenös injizierbaren radioaktiven Diagnosemittels zum Nachweisen einer venösen Thrombose durch nuklearmedizinische Techniken eignen sich alle durch enzymatischen oder nicht-enzymatischen Abbau von menschlichem Plasminogen gewonnenen Fragmente, z.B. das bekannte Neoplasmin, insbesondere Neoplasmin-Val-442 mit einem Molekulargewicht von etwa 38'000.

Menschliches Plasminogen wird mit einer Dialyse gegen Tris-Puffer gereinigt und anschliessend mit Trypsin-Inhibitor und Elastase bei pH 7,8 gespalten. Nach etwa 20 Stunden wird die Reaktion durch Zugabe von Phenylmethylsulfonylfluorid gestoppt. Das Reaktionsgemisch wird vorerst gegen 100 mM Phosphatpuffer dialysiert und nachher mit 0,3 M Phosphatpuffer bei pH 7,8 über eine Lysin-Sepharose-Säule isoliert, wobei Fraktionen zu 4 ml gebildet werden. Die Fraktionen Nr. 9 bis Nr. 27 enthalten Mini- oder Neoplasminogen. Durch Ultrafiltration wird nachher das Volumen je nach der Menge des Rohproduktes auf 4 bis 10 ml reduziert, wobei sich eine Neoplasminogen-Konzentration von etwa 2 mg/ml ergibt. Die Gewinnung von Neoplasminogen ist ausführlich beschrieben in
einer Literaturstelle von James R. Powell und Francis J.
Castellino: "Activation of Human Neo-Plasminogen-Val$_{442}$ by
Urokinase and Streptokinase and a Kinetic Characterization
of Neo-Plasmin-Val$_{442}$", Journal of Biological Chemistry 255
(11) Seiten 5329-5335.

Das erzeugte Neoplasminogen wird z.B. mit dem Isotop I-123
radioaktiv markiert, und zwar zweckmässig nach dem bekannten
Jodogenverfahren, wie es beispielsweise von W.G. Wood,
Chr. Wachter und P.C. Scriba in Fesenius Z. Anal. Chem. 301,
119 (1980), Springer Verlag, beschrieben worden ist. Demgemäss wird z.B. wie folgt vorgegangen:

Eine Lösung von Jodogen in Chloroform wird so hergestellt,
dass 1 mg Jodogen pro 1 ml Lösung resultiert. Diese Lösung
wird in Portionen von 0,2 ml in Fläschchen mit 10 ml Volumen verteilt, wonach das Chloroform mit Stickstoff vertrieben wird. Zum Jodogen gibt man eine Lösung von 1 bis 2 mg
des zuvor gebildeten Neoplasminogens in 1 ml 0,3 M Phosphatpuffer (pH = 7,3), sowie ein Minimagnet-Rührstäbchen
und als letztes 5 bis 8 mCi I-123 in etwa 0,1 ml 0,1 M
Natronlauge. Man lässt 15 Minuten reagieren und kühlt dabei
mit Leitungswasser auf etwa 10$^{o}$C. Am Schluss der Reaktionszeit wird mit Wasser auf 2,5 ml verdünnt und die ganze
Lösung durch ein Millex GV Sterilfilter (0,22 µ) filtriert.

Bei diesem Markierungsverfahren wird das Isotop I-123 kovalent an freie Tyrosingruppen (HO-C$_6$H$_4$-CH$_2$-CH(NH$_2$)COOH)
des Neoplasminogens gebunden und - im Gegensatz zum Stand der Technik - nicht an irgendeiner Stelle des Neoplasminogens lediglich durch Adsorption angeheftet.

Die physikalische Unversehrtheit des radioaktiv markierten
Neoplasminogens lässt sich mittels Poly-Acrylamid-Gel-
Elektrophorese prüfen. Für markiertes und unmarkiertes
Neoplasminogen ergeben sich die gleichen Resultate. Ueber
eine enzymatische Farbreaktion lässt sich ferner die biologische Aktivität des Produktes bestimmen; sie beträgt
üblicherweise etwa 75 %. Schliesslich kann man mittels
Dünnschicht Chromatographie auch den Anteil an freiem $I^-$
überprüfen.

Aus dem mit I-123 markierten Neoplasminogen wird eine Vorstufe des intravenös injizierbaren Diagnosemittels hergestellt und in Patientendosen aufgeteilt, wobei jede Patientendosis vorzugsweise die folgende Spezifikation aufweist: 250 µg $^{123}$I-Neoplasminogen in 200 µl 0,1 M Phosphatpuffer (pH = 7,8), Aktivität 200 µCi, steril und
pyrogenfrei. Diese Dosis wird etwa 15 Minuten vor der intravenösen Injektion mit 3000 E Urokinase biologisch aktiviert,
wodurch eine Spaltung des $^{123}$I-Neoplasminogens eingeleitet
und die Bildung von radioaktiv markiertem Neoplasmin herbeigeführt wird.

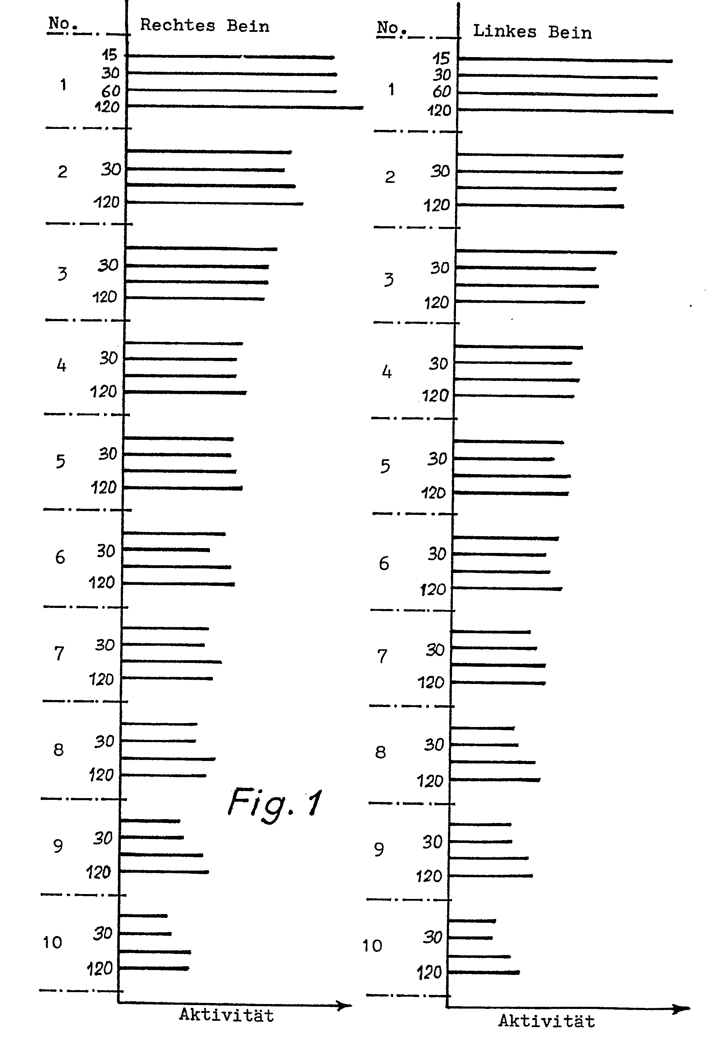

Mit dem auf die beschriebene Weise vorbereiteten Diagnosemittel wurden die nachstehenden Tests an den unteren Extremitäten von fünf gesunden Probanden und neun Patienten mit klinischem Verdacht auf eine tiefe venöse Thrombose durchgeführt. Zur Vermeidung einer selektiven Anreicherung von Jod
in der Schilddrüse wurden am Tag der Untersuchung 3 x 10
Tropfen Kaliumjodid-Lösung verordnet. 200 µCi $^{123}$I-Neoplasminogen wurden mit 3000 E Urokinase aktiviert und 15 Minuten
später den Probanden und den Patienten intravenös injiziert.
Mittels eines transportablen Pittman Monitors wurde die
Oberflächenaktivität an 10 bzw. 13 gleichmässig verteilten
Messpunkten entlang den Venen des rechten und des linken Beines jedes Probanden bzw. Patienten gemessen, und zwar jeweils
nach Ablauf von 15, 30, 60 und 120 Minuten nach der Injektion des Diagnosemittels. Die Messresultate sind in den Fig. 1
und 2 graphisch aufgezeichnet.

Fig. 1 bezieht sich auf die Messresultate an einem gesunden
Probanden. Die Oberflächenaktivitäten wurden an zehn Messpunkten ermittelt, die in Fig. 1 mit No. 1 bis 10 bezeichnet
sind, wobei der Messpunkt No. 1 jeweils beim Hüftgelenk und
der Messpunkt No. 10 jeweils beim Fussknöchel lag. Die Aktivitätsmessungen am rechten Bein und am linken Bein erfolgten
jeweils mit der gleichen Messapparatur, wobei immer die gleiche Masseinheit zur Anwendung gelangte. Es zeigte sich, dass
jeweils an zwei einander entsprechenden Messpunkten am rechten und am linken Bein annähernd gleich hohe Aktivitäten vorliegen. Als Kriterium wurde jeweils der Quotient aus den gemessenen Aktivitäten an den einander entsprechenden Messpunkten am rechten und am linken Bein ermittelt. Dieser Quotient
ergab sich bei allen fünf gesunden Probanden zu 1,042 $\pm$ 0,9
bei Extremwerten zwischen 0,90 und 1,20.

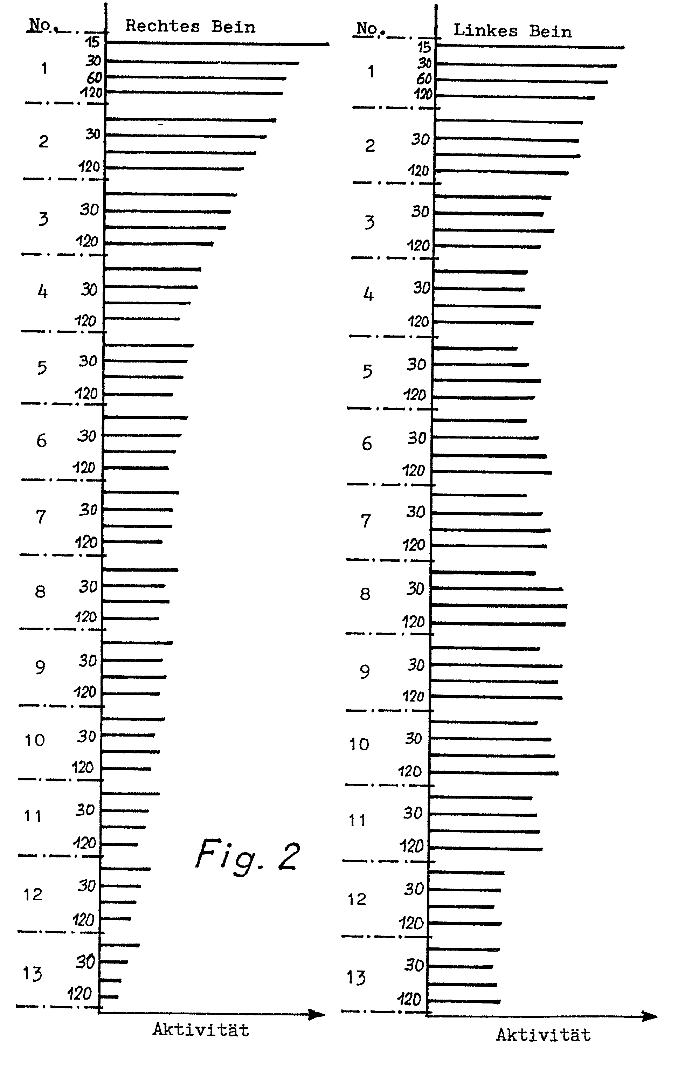

Fig. 2 bezieht sich auf die Messresultate an einem Patienten
mit einer tiefen venösen Thrombose. Die Oberflächenaktivitäten
wurden an dreizehn Messpunkten ermittelt, die in Fig. 2 mit
No. 1 bis No. 13 bezeichnet sind, wobei der Messpunkt No. 1
jeweils beim Hüftgelenk und der Messpunkt No. 13 jeweils beim
Fussknöchel lag. Die Aktivitätsmessungen am rechten Bein und am linken Bein erfolgten jeweils mit der gleichen Messapparatur, wobei immer gleiche Masseinheiten zur Anwendung gelangten. Es wurde wieder der Quotient aus den gemessenen Aktivitäten an zwei einander entsprechenden Messpunkten am rechten und am linken Bein ermittelt. Dabei zeigte sich, dass bei
den Messpunkten No. 6 bis 13 der resultierende Quotient jeweils
beträchtlich von 1 abwich, da an diesen Messpunkten am linken Bein des Patienten jeweils eine merklich höhere Radioaktivität vorlag als an den entsprechenden Messpunkten am
rechten Bein. Daraus lässt sich schliessen, dass im linken
Bein dieses Patienten im Bereich vom Fussknöchel bis oberhalb des Knies eine Thrombose vorlag. Aehnliche Ergebnisse
zeigten sich bei sieben weiteren der insgesamt neun untersuchten Patienten. Bei allen acht Patienten mit einem positiven Diagnosetest war der Quotient aus den gemessenen Aktivitäten an einigen der einander entsprechenden Messpunkte
am rechten und am linken Bein entweder kleiner als 0,9 oder
grösser als 1,2. Der erwähnte Quotient ist daher als diagnostisches Kriterium brauchbar, wobei die kritischen Grenzwerte etwa bei 0,9 bzw. 1,2 liegen.

Die Ergebnisse der beschriebenen Diagnosetests wurden mit den
Resultaten anderer Diagnoseverfahren verglichen und sind in
der folgenden Tabelle zusammengestellt.

### T a b e l l e

| | |
|---|---|
| Klinik: | 9 Patienten positiv |
| Neoplasmintest: | 8 Patienten positiv, 1 negativ |
| Phlebographietest: | 8 Patienten positiv, 1 negativ |
| Dopplersonnographietest: | 6 Patienten positiv, 3 negativ |

Bei dem einzigen Patienten mit negativem Resultat des erfindungsgemässen Neoplasmintests konnte auch phlebographisch keine Thrombose nachgewiesen werden.

Auf Grund dieser Befunde kann festgestellt werden, dass durch eine nicht invasive Untersuchung mit dem oben beschriebenen Diagnosemittel, das ein mit dem Gammastrahler I-123 markiertes Neoplasmin enthält, ebenso treffsicher wie durch einen invasiven Phlebographietest das Vorliegen oder die Abwesenheit einer venösen Thrombose diagnostiziert werden kann. Im Gegensatz zu dem ebenfalls nicht invasiven Fibrinogentest verlangt das neue Diagnosemittel nur eine vergleichsweise kurze Wartezeit von 15 bis 30 Minuten zwischen der intravenösen Injektion des Diagnosemittels und den nuklearmedizinischen Aktivitätstests, wie aus Fig. 2 deutlich erkennbar ist. Gegenüber Untersuchungen mittels Dopplersonographie scheint der beschriebene Diagnosetest mit intravenös injiziertem, durch I-123 radioaktiv markiertem Neoplasmin insbesondere im Bereich der Unterschenkel überlegen zu sein.

Da in dem beschriebenen Diagnosemittel das Isotop I-123 kovalent an Tyrosingruppen des Neoplasmins gebunden ist, wird dieses Diagnosemittel im menschlichen Organismus als einheitliche und eindeutig definierte Substanz genau gleich abgebaut wie natürliches menschliches Plasmin, das am Ort einer Thrombose von selbst gebildet wird. Deshalb hat dieses Diagnosemittel keine unerwarteten und gegebenenfalls gesundheitsstörenden Nebenwirkungen zur Folge.

Beispiel 2:
Ein intravenös injizierbares Diagnosemittel in Form eines radioaktiven Plasminogenfragmentes, das anstatt mit I-123 mit einem anderen radioaktiven Halogen aus der Gruppe I-131, I-124, Br-77 und F-18 markiert ist, lässt sich gleich oder analog zum Beispiel 1 herstellen, wobei sich die gleichen Wirkungen und Vorteile des Diagnosemittels ergeben. Insbesondere ist zu erwähnen, dass auch in diesen Fällen das radioaktive Isotop jeweils kovalent an Tyrosingruppen des Plasminogenfragmentes gebunden sind. Das Isotop I-131 ist wie I-123 ein Gammastrahler, wogegen die Isotopen I-124, F-18 und Br-77 Positronenstrahler sind.

Beispiel 3:

Menschliches Neoplasminogen wird zubereitet,wie im Beispiel 1
ausführlich beschrieben ist.

Das Neoplasminogen wird mit dem radioaktiven Isotop Tc-99m
derart markiert, dass letzteres mit Hilfe eines bifunktionellen Chelators (auch bifunktioneller Ligand oder Brücken-
Ligand genannt) an freie Aminogruppen des Neoplasminogens
kovalent gebunden wird. Als bifunktioneller Chelator eignet
sich z.B. eine aktivierte Carbonsäure (z.B. ein gemischtes
Anhydrid oder ein zyklisches Anhydrid).

Die Markierung geschieht beispielsweise wie folgt:
Als bifunktioneller Chelator dienendes Dianhydrid von
Diäthylentriaminpentaacetat (DTPA) wird nach dem bekannten Verfahren von Eckelmann (J. Pharm. Sciencies Vo. 64
(1975) p. 704) hergestellt. In Dimethylformamid wird
eine gesättigte Lösung dieser Substanz vorbereitet. 0,1 ml
dieser Lösung gibt man unter Rühren zu 2 bis 5 mg Neoplasminogen, das in 0,5 ml 0,1 M Phosphatpuffer (pH 7,5) gelöst ist.
Nach einer Minute Reaktionszeit bei Zimmertemperatur trennt
man das Protein über eine G25-Sephadex-Säule vom freien DTPA,
das in diesem Zeitpunkt vollständig hydrolisiert ist. Die Markierung geschieht mit einem Hilfskomplex durch Austauschmarkierung. Als erstes wird dazu ein kommerziell erhältlicher
MDP-Kit (d.h. eine vorbereitete Mischung aus Methylendiphosphonat und $SnCl_2$, lyophilisiert) in bekannter und üblicher
Weise mittels Tc-99m-Pertechnetat markiert. Das so markierte
MDP wird mit dem vorher zubereiteten Gemisch aus Neoplasminogen und DTPA gemischt und während etwa 1 Stunde bei Raumtemperatur reagieren gelassen.

Die chromatographisch bestimmte Ausbeute beträgt 70 ± 20 %
(Celluloseplatten CEL 300 und physiologische Kochsalzlösung
als Eluens), und die mit Hilfe des chromogenen Substrates bestimmte biologische Aktivität liegt bei 75 ± 15 %.

Durch dieses Markierungsverfahren wird das Isotop Tc-99m mittels des bifunktionellen Chelators an eine freie Aminogruppe von Lysin in der Peptidkette des Neoplasminogens gebunden unter Ausbildung eines Amids, so dass ein eindeutig definiertes Produkt resultiert, im Gegensatz zum Stand der Technik, gemäss welchem Tc-99m in Form von kolloidalem $TcO_2$ lediglich durch Adsorption an irgendeiner unbestimmten Stelle des Neoplasminogens angeheftet worden ist.

Die weitere Verwendung des mit Tc-99m markierten Neoplasminogens ist gleich,wie im Beispiel 1 beschrieben wurde.

Beispiel 4:
Prinzipiell gleich oder analog zum Beispiel 3 lässt sich ein radioaktives Neoplasminogen bzw. Neoplasmin anstatt mit Tc-99m mit einem anderen radioaktiven Metallion aus der Gruppe In-111 und Ru-97 markieren, wobei sich die gleichen vorteilhaften Wirkungen des Diagnosemittels wie beim Beispiel 1 ergeben. Insbesondere ist hervorzuheben, dass auch in diesen Fällen das radioaktive Metallion jeweils mittels eines bifunktionellen Chelators oder Brücken-Liganden an freie Aminogruppen des Neoplasminogens kovalent gebunden werden. Die Isotopen Tc-99m und Ru-97 sind Gammastrahler.

Beispiel 5:
Die Markierung von Neoplasminogen mit einem radioaktiven Metallion, wie Tc-99m, Ru-97 oder In-111, kann alternativ mittels Azokupplung an freie Tyrosingruppen des Neoplasminogens erfolgen, wobei wieder die gleichen vorteilhaften Wirkungen des als Diagnosemittel verwendeten radioaktiven Neoplasmins erzielt werden.

Patentansprüche

1. Intravenös injizierbares radioaktives Diagnosemittel zum Nachweisen einer venösen Thrombose durch nuklearmedizinische Techniken, welches Diagnosemittel ein mit einem radioaktiven Atom markiertes Fragment von menschlichem Plasminogen aufweist, dadurch gekennzeichnet, dass als Gamma- oder Positronenstrahler ein radioaktives Halogen kovalent an freie Tyrosingruppen des Plasminogenfragmentes gebunden ist oder ein radioaktives Metallion mittels eines bifunktionellen Chelators kovalent an freie Aminogruppen des Plasminogenfragmentes oder mittels Azokupplung an freie Tyrosingruppen des Plasminogenfragmentes gebunden ist.

2. Diagnosemittel nach Anspruch 1, dadurch gekennzeichnet, dass das Plasminogenfragment mit einem Gammastrahler mit einer Energie im Bereich von 80 bis 400 keV markiert ist.

3. Diagnosemittel nach Anspruch 2, dadurch gekennzeichnet, dass der an das Plasminogenfragment gebundene Gammastrahler I-123, I-131, In-111, Ru-97 oder Tc-99m ist.

4. Diagnosemittel nach Anspruch 1, dadurch gekennzeichnet, dass der an das Plasminogenfragment gebundene Positronenstrahler F-18, I-124 oder Br-77 ist.

5. Diagnosemittel nach einem der Ansprüche 1 bis 4, dadurch gekennzeichnet, dass das Plasminogenfragment Neoplasmin-Val-442 mit einem Molekulargewicht von etwa 38'000 ist.

6. Verfahren zur Herstellung eines intravenös injizierbaren radioaktiven Diagnosemittels zum Nachweisen einer venösen Thrombose durch nuklearmedizinische Techniken, nach einem der Ansprüche 1 bis 5, dadurch gekennzeichnet, dass menschliches Plasminogen mit Elastase gespalten und aus dem Reaktionsgemisch Neoplasminogen durch Chromatographie gewonnen wird, dass ein radioaktives Halogen kovalent an freie Tyrosingruppen des Neoplasminogens gebunden wird oder ein radioaktives Metallion mittels eines bifunktionellen Chelators kovalent an freie Aminogruppen des Neoplasminogens oder mittels Azokupplung an freie Tyrosingruppen des Neoplasminogens gebunden wird, und dass das so mit einem Gamma- oder Positronenstrahler markierte Neoplasminogen vor der intravenösen Injektion mit Urokinase weiter gespalten wird zur Bildung von radioaktiv markiertem, intravenös injizierbarem Neoplasmin.

7. Verfahren nach Anspruch 6, dadurch gekennzeichnet, dass man Patientendosen, die je mindestens 200 µCi des radioaktiv markierten Neoplasminogens in mindestens 200 µl 0,1 M Phosphatpuffer mit pH 7,8 enthalten, bildet und einer solchen Dosis jeweils etwa 15 Minuten vor der intravenösen Injektion etwa 3000 E Urokinase zufügt.

8. Präparat zur Herstellung eines intravenös injizierbaren radioaktiv markierten Fragmentes von menschlichem Plasminogen zum Nachweisen einer venösen Thrombose durch nuklearmedizinische Techniken, dadurch gekennzeichnet, dass das vor der intravenösen Injektion mit Urokinase zu behandelnde Präparat mindestens 200 µCi von mit einem Gamma- oder Positronenstrahler markiertem menschlichem Neoplasminogen enthält, bei welchem ein radioaktives Halogen kovalent an freie Tyrosingruppen des Neoplasminogens gebunden ist oder ein radioaktives Metallion mittels eines bifunktionellen Chelators kovalent an freie Aminogruppen des Neoplasminogens oder mittels Azokupplung an freie Tyrosingruppen des Neoplasminogens gebunden ist.

9. Präparat nach Anspruch 8, dadurch gekennzeichnet, dass das mit einem Gamma- oder Positronenstrahler markierte

- 3 -    0170031

menschliche Neoplasminogen in mindestens 200 µl 0,1 M Phosphatpuffer mit pH 7,8 enthalten ist.

10. Präparat nach Anspruch 8 oder 9, dadurch gekennzeichnet, dass der an das Neoplasminogen gebundene Gammastrahler I-123, I-131, In-111, Ru-97 oder Tc-99m ist.

11. Präparat nach Anspruch 10, dadurch gekennzeichnet, dass der Gammastrahler eine Energie im Bereich von 80 bis 400 keV aufweist.

12. Präparat nach Anspruch 8 oder 9, dadurch gekennzeichnet, dass der an das Neoplasminogen gebundene Positronenstrahler F-18, I-124 oder Br-77 ist.

Fig. 1

2/2

0170031

No. — Rechtes Bein

No. — Linkes Bein

Fig. 2

Aktivität

Aktivität

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | GB-A-2 090 599 (NOVO INDUSTRI) * Beispiel 3; Seite 2, Zeilen 49-62 * | 1-12 | A 61 K 49/02 |
| Y | CHEMICAL ABSTRACTS, Band 90, Nr. 25, 18. Juni 1979, Seite 232, Nr. 199568c, Columbus, Ohio, US; U. CHRISTENSEN et al.: "Enzymic properties of the neo-plasmin-Val-442 (miniplasmin)" & BIOCHIM. BIOPHYS. ACTA 1979, 567(2), 472-81 * Insgesamt * | 1-12 | |
| Y | CHEMICAL ABSTRACTS, Band 95, Nr. 9, 31. August 1981, Seite 509, Nr. 77735n, Columbus, Ohio, US; L.A. MOROZ: "Mini-plasminogen: a mechanism for leukocyte modulation of plasminogen activation by urokinase" & BLOOD 1981, 58(1), 97-104 * Insgesamt * | 1-12 | RECHERCHIERTE SACHGEBIETE (Int Cl 4) A 61 K |

-/-

Der vorliegende Recherchenbericht wurde für alle Patentansprüche erstellt

| Recherchenort | Abschlußdatum der Recherche | Prüfer |
|---|---|---|
| DEN HAAG | 22-10-1985 | RYCKEBOSCH A.O.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| D,Y | CHEMICAL ABSTRACTS, Band 93, Nr. 7, 18. August 1980, Seite 431, Nr. 65173y, Columbus, Ohio, US; W.G. WOOD et al.: "Comparison of iodine-125 labeling of polypeptides using chloramine-T and 1,3,4,6-tetrachloro-3alpha,6alpha-diphenylglycoluril (Iodogen(R)) with subsequent separation of iodide and labeled protein on cation-exchange dextran" & FRESENIUS' Z. ANAL. CHEM. 1980, 301(2), 301(2), 119 * Insgesamt * | 1-12 | |
| | --- | | |
| Y | EP-A-0 035 765 (NIHON MEDI-PHYSICS CO. LTD.) * Ansprüche; Seite 2, Zeile 14 - Seite 3, Zeile 22 * | 1-12 | RECHERCHIERTE SACHGEBIETE (Int. Cl.4) |
| | --- | | |
| Y | EP-A-0 038 546 (THE MASSACHUSETTS GENERAL HOSPITAL) * Ansprüche 1-4,10,14; Seite 3, Zeilen 15-32; Seite 2, Zeilen 12-17 * | 1-12 | |
| | --- | | |
| A | US-A-4 305 922 (B.A. RHODES) * Anspruch 1 * | 1 | |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 22-10-1985 | RYCKEBOSCH A.O.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82